# EUROPÄISCHE PATENTSCHRIFT

(11) **EP 0 786 975 B1**
(45) Veröffentlichungstag und Bekanntmachung des Hinweises auf die Patenterteilung: **22.12.1999**
(21) Anmeldenummer: 95934033.2
(22) Anmeldetag: 20.10.1995
(51) Int. Cl.: A61F 2/18, A61C 8/00, A61C 9/00

(54) **IMPLANTAT-SYSTEM FÜR EXTRA-ORALE APPLIKATIONEN UND DIE ORTHODONTIE**
IMPLANT SYSTEM FOR EXTRA-ORAL APPLICATIONS AND ORTHODONTICS
SYSTEME D'IMPLANT POUR APPLICATIONS EXTRA-ORALES ET L'ORTHODONTIE

(30) Priorität: 21.10.1994 CH 316594
(43) Veröffentlichungstag der Anmeldung: 06.08.1997
(73) Patentinhaber: INSTITUT STRAUMANN AG, 4437 Waldenburg (CH)
(72) Erfinder: ASIKAINEN, Pekka, FIN-70800 Kuopio (FI); SUTTER, Franz, CH-4435 Niederdorf (CH); MUNDWILER, Ulrich, CH-4456 Tenniken (CH); MERZ, Beat, CH-5004 Aarau (CH); WEHRBEIN, Heiner, D-52064 Aachen (DE); GLATZMAIER, Jürgen, D-80637 München (DE); HAUSMANN, Dieter, D-49086 Osnabrück (DE); HÜSKENS, Christoph, CH-4410 Liestal (CH)
(74) Vertreter: Ullrich, Gerhard, Dr.
(86) Internationale Anmeldenummer: CH9500246
(87) Internationale Veröffentlichungsnummer: WO9612451

(56) Entgegenhaltungen:
- EP-A- 0 497 082
- WO-A-93/20774
- US-A- 5 007 835
- US-A- 5 071 345

## Beschreibung

Die Erfindung betrifft ein Implantat-System für die Verankerung von orthodontischen Anordnungen im oralen Bereich zur Erzielung von Zahnstellungskorrekturen. Das Implantat-System besteht aus einem orthodontischen Implantat (verkürzt als Orthoimplantat bezeichnet) und einer Klemmkappe.

### Stand der Technik

Ein Implantat-System wie im Oberbegriff von Anspruch 1 angegeben, ist aus der EP-A-0 497 082 bekannt.

Zur Erzielung von bestimmten Zahnstellungskorrekturen ist es bisher nötig, dass der Patient einen Drahtbügel, den sogenannten Headgear, um den Kopf trägt. Diese Apparatur ist umständlich, störend und daher unbeliebt. Darüber hinaus geht vom Headgear eine erhebliche Verletzungsgefahr aus. Es sind damit ernsthafte Kopfverletzungen bekanntgeworden, die durch das Losschnellen der unter Spannung stehenden Drahtbügel verursacht wurden.

Lediglich wenn der Patient über ein ausreichendes Knochenangebot verfügt und im Mundraum genügend interokklusaler Abstand zur Verfügung steht, kann man bisher im Kieferkammbereich Implantate als Verankerungspunkt setzen. Diese Implantate sind jedoch relativ gross und kommen daher z.B. für Jugendliche weniger in Betracht (vgl. Wehrbein et al., in Dtsch Zahnärztl Z 1993, 48, S.191 ff.).

Zusammenfassend ist festzustellen, dass die bis dato vorhandenen orthodontischen Implantat-Systeme und orthodontischen Apparaturen allesamt hinsichtlich ihrer Implantation und Dimension, ihrer Tragfähigkeit und Zuverlässigkeit sowie ihrer universellen Einsetzbarkeit nicht als optimal bezeichnet werden können.

### Aufgabe der Erfindung

Die Erfindung hat sich daher die Aufgabe gestellt, ein vervollkommnetes orthodontisches Implantat-System zu schaffen. Mit diesem System soll das Leistungsverhältnis zwischen Bauteilgrösse und Tragfähigkeit verbessert werden. Insbesondere gilt es, die Infektionsgefahren zu minimieren und die Primärstabilität deutlich zu steigern, so dass von Anfang an - also sogleich nach dem Implantieren - eine möglichst hohe Belastbarkeit bzw. eine reduzierte Einheilphase gegeben ist. Die Erfindung verfolgt die Zielstellung, den Anwendungsbereich von Implantaten in der Orthodontie zu erweitern und dazu komplementäre Bauteile sowie zugehörige orthodontische Anordnungen vorzuschlagen.

### Übersicht über die Erfindung

Zum Implantat-System gehört zunächst ein Orthoimplantat, welches einen Gewindeteil mit einem Aussengewinde besitzt, das zum Einsetzen in einen Knochen bestimmt ist. Am Gewindeteil sind Gewindezähne und durch Schneidausnehmungen gebildete Schnittkanten vorhanden. In seiner Kopfzone ist das Orthoimplantat komplementär zu einer aufsetz- und fixierbaren Klemmkappe gestaltet. Auf der dem Orthoimplantat zugewandten Seite weist die Klemmkappe eine tangentiale Drahtnut auf. Zwischen dem Orthoimplantat und der Klemmkappe lässt sich ein zugeführter orthodontischer Draht einklemmen, wobei die Klemmkappe mit einer Okklusalschraube auf das Orthoimplantat geschraubt ist. Der orthodontische Draht, welcher sich hin zu einem zu verschiebenden Zahn oder zu einer zu verschiebenden Zahngruppe erstreckt, ist einseitig oder beiderseits abgehend unter der Klemmkappe fixierbar.

Ein Schraubendreher ist insbesondere zum Auf- und Abschrauben sowie Positionieren der Klemmkappe konzipiert. Vorgesehen ist eine im Verhältnis zu einem Ratschenkopf drehbare und auf einem Schraubendreherschaft verschiebbare, jedoch auch fixierbare Hülse. Die Hülse nimmt an ihrer Eintrittsseite die Klemmkappe mit eingesetzter Okklusalschraube und zugewandter Oberseite auf. Axial durch die Hülse ist der Ausnehmung in der Okklusalschraube ein Schraubendreherkopf zuführbar, wobei der Schraubendreherkopf und die Ausnehmung zueinander komplementäre Konturen besitzen.

Durch das erfindungsgemässe Implantat-System mit dem Orthoimplantat und der Klemmkappe erweitern sich die Anwendungsmöglichkeiten in der Orthodontie. Die Orthoimplantate können retromolar oder palatinal gesetzt werden, an denen sich justierte, orthodontische Drähte fixieren lassen, die direkt oder indirekt zu Zahnstellungskorrekturen nutzbar sind. Ein spezieller Kreuzschraubendreher ermöglicht ein effizientes Handling und ein sicheres Fassen, Justieren sowie Verschrauben der auf die Orthoimplantate aufzusetzenden Klemmkappen.

### Kurzbeschreibung der beigefügten Zeichnungen

Es zeigen:
- Figur 1A:: ein orthodontisches Implantat als Teilschnitt;
- Figur 1B:: das Aussengewinde des orthodontischen Implantats gemäss Figur 1A im Detail als vergrösserter Schnitt;
- Figur 1C:: den Aussengewindeschaft des orthodontischen Implantats gemäss Figur 1A als Ansicht von unten;
- Figur 1D:: den Schnittvorgang des orthodontischen Implantats gemäss Figur 1A im Knochen als Prinzipdarstellung;
- Figur 2A:: eine Klemmkappe für das orthodontische Implantat gemäss Figur 1A als Seitenansicht im Schnitt;
- Figur 2B:: die Klemmkappe gemäss Figur 2A als Ansicht von unten;
- Figur 3:: eine Okklusalschraube;
- Figur 4:: ein orthodontisches Implantat gemäss Figur 1A im Knochen, komplett zusammengebaut, mit eingeklemmtem, orthodontischem Draht, aufgesetzter Klemmkappe gemäss Figur 2A und eingeschraubter Okklusalschraube gemäss Figur 3 als Teilschnitt;

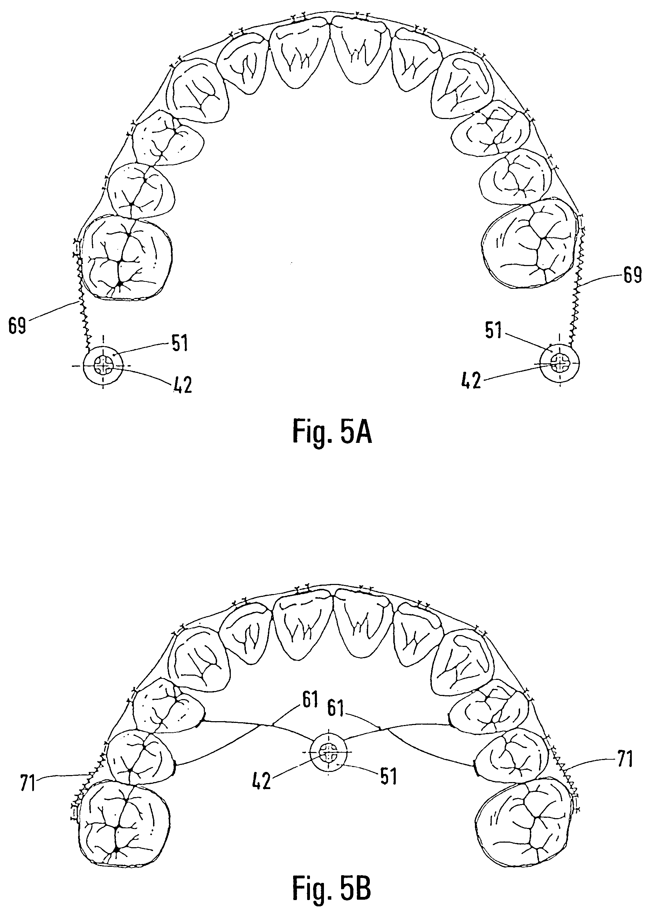

- Figur 5A:: das orthodontische Implantat gemäss Figur 1A im Oberkiefer retromolar gesetzt;
- Figur 5B:: das orthodontische Implantat palatinal gesetzt und an Prämolaren angreifend;

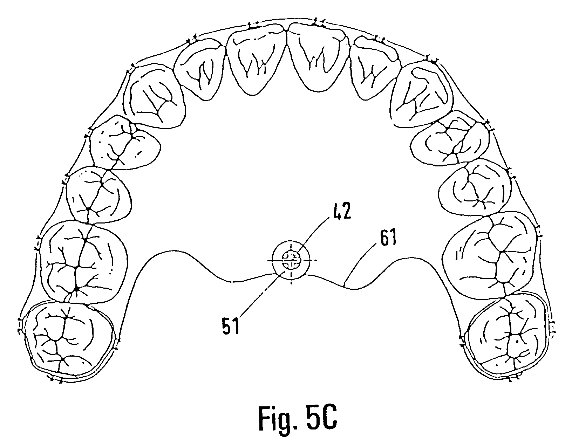

- Figur 5C:: das orthodontische Implantat am zweiten Molar angreifend;

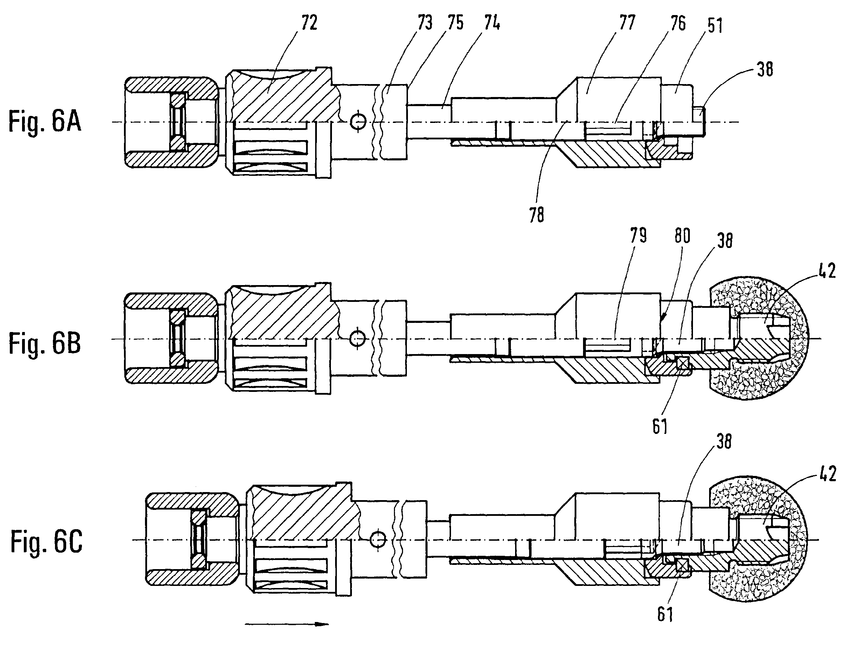

- Figur 6A:: einen Schraubendreher zum Aufsetzen der Klemmkappe gemäss Figur 2A und der Okklusalschraube gemäss Figur 3;
- Figur 6B:: den Schraubendreher gemäss Figur 6A mit fixierter Haltehülse; und
- Figur 6C:: den Schraubendreher gemäss Figur 6A mit aktiviertem Ratschenkopf.

### Ausführungsbeispiele

Anhand der beiliegenden Zeichnungen erfolgt nachstehend die detaillierte Beschreibung eines Ausführungsbeispiels zum erfindungsgemässen Implantat-System und zum Schraubendreher.

Für die gesamte weitere Beschreibung gilt folgende Festlegung. Sind in einer Figur zum Zweck zeichnerischer Eindeutigkeit Bezugsziffern enthalten, aber im unmittelbar zugehörigen Beschreibungstext nicht erläutert, so wird auf deren Erwähnung in vorangehenden oder nachfolgenden Figurenbeschreibungen Bezug genommen. Im Interesse der Übersichtlichkeit wird auf die wiederholte Bezeichnung von Bauteilen in weiteren Figuren zumeist verzichtet, sofern zeichnerisch eindeutig erkennbar ist, dass es sich um "wiederkehrende" Bauteile handelt.

### Figur 1A

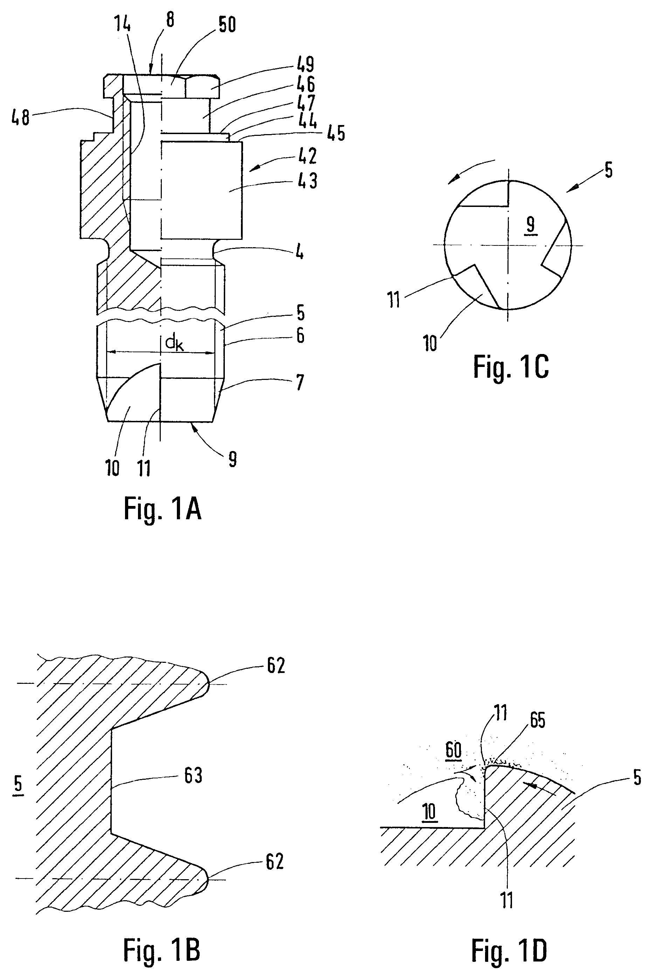

Das schraubenähnliche Orthoimplantat **42** besteht aus einem etwa mittig angeordnetem Schaftstück **4** mit dem Kerndurchmesser **dk** des darunter liegenden Gewindeteiles **5**, auf welchem ein spezielles Aussengewinde **6** vorgesehen ist (s. Figuren 1B bis 1D). An das Gewindeteil **5** schliesst sich eine konische Eindrehhilfe **7** an. Das Orthoimplantat **42** ist nach oben durch die Deckfläche **8** und nach unten hin durch die Basisfläche **9** an der Eindrehhilfe **7** begrenzt. Zur Basisfläche **9** hin verjüngt sich die Eindrehhilfe **7** konisch. Das Aussengewinde **6** läuft auf der Eindrehhilfe **7** aus. Von der Basisfläche **9** erstrecken sich drei peripher an der Eindrehhilfe **7** angeordnete und axial bis in das Gewindeteil **5** reichende, nutenartige Schneidausnehmungen **10**. Die hierdurch entstehenden vertikalen Kanten dienen als Schnittkanten **11** im Sinne eines selbstschneidenden Gewindes.

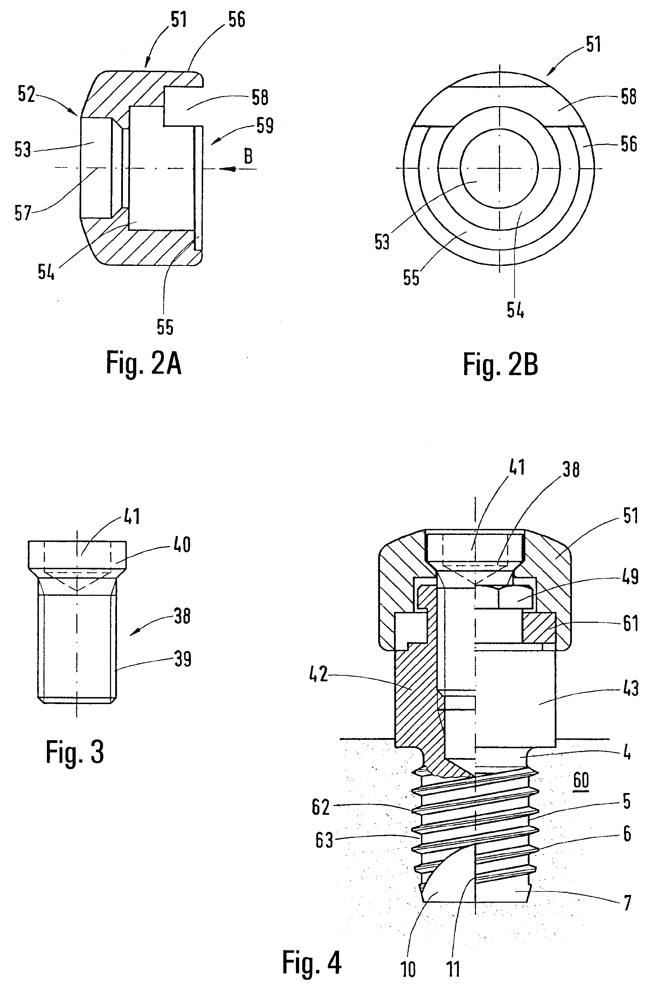

Über dem Schaftstück **4** ist ein Zylinderstück **43** angeordnet, auf welchem ein weiteres, im Durchmesser reduziertes Zylinderstück **44** sitzt. Hierdurch entsteht ein Ringabsatz **45**. Dieser Ringabsatz **45** dient der Aspirationssicherung beim Einsetzen des Orthoimplantates **42**; dort greift ein Kunststoffring am Eindrehwerkzeug mit leichtem Untermass. Über dem reduzierten Zylinderstück **44** folgt ein deutlich im Durchmesser reduziertes Kopfzylinderstück **46**. Zwischen den Zylinderstücken **44** und **46** entsteht ein grösserer Auflageabsatz **47**. Dieser Auflageabsatz **47** bildet zusammen mit dem Kopfzylinderstück **46** das Widerlager **48** für den orthodontischen Draht, der mit einer aufschraubbaren Klemmkappe fixiert wird (s. Figuren 2A bis 4). Den oberen Abschluss des Orthoimplantats **42** bildet ein Schraubenmutterstück **49** mit einem Achtkant **50**, an welches ein passendes Werkzeug zum Eindrehen des Orthoimplantats **42** ansetzt. Von der Deckfläche **8** erstreckt sich axial ein Innengewinde-Sackloch **14**, das die Okklusalschraube zum Aufschrauben und Fixieren der Klemmkappe aufnimmt.

### Figuren 1B bis 1D

Mit Bezug auf diese Figuren werden nun die Beschaffenheit des selbstschneidenden Aussengewindes **6** am Gewindeteil **5** des Orthoimplantates **42** sowie der Eindrehvorgang in den Knochen **60** beschrieben. Die Oberfläche des Orthoimplantats **42**, zumindest jedoch deren Gewindeteile **5**, sind durch Sandstrahlen und anschliessendes Ätzen aufgerauht. Infolge dieser Behandlung sind sowohl die Gewindezähne **62** als auch die Schnittkanten **11** abgerundet. Zwischen den Gewindezähnen **62** befinden sich genügend breite Gewindegründe **63**, so dass die Sandstrahlkörner die Gewindegründe **63** erreichen. Beim Schneidvorgang wird Knochenmaterial vom Knochen **60** durch die abgerundeten Schnittkanten **11** abgetragen und in die Schneidausnehmungen **10** geschoben. Durch die Abrundungen an den Schnittkanten **11** erfolgt jedoch kein konsequentes Abschaben von Knochenmaterial, sondern ein Teil des Knochenmaterials wird abgedrängt und am Rande des im Knochen 60 vorgebohrten Sacklochs verdichtet.

### Figuren 2A und 2B

Die Klemmkappe **51** besitzt haubenförmige Gestalt mit komplementären Innenkonturen zum Kopfbereich des Orthoimplantates **42**. Auf der Oberseite **52** der Klemmkappe **51** ist eine eingesenkte Bohrung **53** zur Aufnahme des Schraubenkopfes **40** der Okklusalschraube **38** vorgesehen. Unter der Bohrung **53** befindet sich die Ausnehmung **54** zur Einbettung des Schraubenmutterstückes **49**. Die Ausnehmung **54** kann entweder - wie dargestellt - zylindrisch glatt sein, wodurch sich die Klemmkappe **51** axial zum Achtkant **50** beliebig drehen lässt, oder sie kann facetiert sein - z.B. als 24-Kant -, wodurch ein Formschluss mit dem Achtkant **50** erreicht wird, der ein Verdrehen der Klemmkappe **51** gegen das Orthoimplantat **42** im festgeschraubten Zustand verhindert. Unter der Ausnehmung **54** folgt die Klemmausnehmung **55**, die auf den Durchmesser des Zylinderstückes **43** erweitert ist. Seitlich umgibt die Klemmkappe **51** ein Kappenmantel **56**. Ausserhalb der Kappenmittelachse **57**, quasi peripher, durchzieht den Kappenmantel **56** eine Drahtnut **58**, welche sich von der Kappenunterseite **59** bis zur Ausnehmung **54** erstreckt.

### Figur 3

Eine Okklusalschraube **38** dient zum Fixieren der Klemmkappe auf dem Orthoimplantat (s. Figur 4). Die Okklusalschraube **38** besitzt einen Gewindeschaft **39** und einen Schraubenkopf **40**. In letztgenanntem ist eine konturierte, sacklochförmige Ausnehmung **41** für den Eingriff eines Schraubendrehers mit komplementärer Spitze vorgesehen.

### Figur 4

Im Knochen **60** sitzt das mit der Klemmkappe **51** versehene Orthoimplantat **42,** wobei die Klemmkappe **51** mit einer Okklusalschraube **38** fixiert ist. Durch die Drahtnut **58** eingeführt und unter der Klemmkappe **51** eingequetscht ist ein positionierter, orthodontischer Draht **61**. Der Draht **61** kann in beliebigem Winkel bzw. in vorgegebenen Winkelschritten positioniert und gehalten werden, je nach Verwendung einer glatten oder facetierten Kontur der in der Klemmkappe **51** vorgesehenen Ausnehmung **54**. Die Drahtnut **58** muss vor dem Fixieren der Klemmkappe **51** nur entsprechend ausgerichtet werden.

### Figur 5A

Gemäss einem ersten Anwendungsfall im Oberkiefer ist retromolar rechts und links je ein Orthoimplantat **42** gesetzt. Am Orthoimplantat **42**, eingeklemmt unter der Klemmkappe **51**, ist eine Zugfeder **69** angebracht, die andererseits am 1. Molar angreift. Auf diese Weise wird der Molar sukzessive distal verschoben. Führt man eine so gerichtete Verschiebung etappenweise auch mit weiteren Zähnen durch, so erzielt man die gewünschte Auflockerung bzw. Korrektur in der Zahnreihe.

### Figur 5B

Gemäss einem weiteren Anwendungsfall im Oberkiefer ist ein Orthoimplantat **42** palatinal - d.h. auf der Mittellinie des kortikalen Gaumenknochens in die anatomische Struktur der sutura palatina mediana - eingesetzt. Mittels der Klemmkappe **51** justiert man orthodontische Drähte **61**, die jeweils auf die beiden Prämolare zulaufen und daran fixiert sind. Vom Orthoimplantat **42** geht nach jeder Seite nur ein Draht **61** ab, der sich vor Erreichen der Prämolare Y-artig aufspaltet. Auf diese Weise hat man eine Blockbildung erreicht und die Prämolaren quasi als Pfosten gestaltet. An der Aussenseite des 2. Prämolaren fixiert man eine Druckfeder **71**, die andererseits am 1. Molar befestigt ist. Hierdurch wird dieser Molar sukzessive distal weggeschoben, während der Block aus Prämolaren und Orthoimplantat unverrückbar ist. Mit gleicher Methode können bei Bedarf weitere Zähne verschoben werden.

### Figur 5C

Praktizierbar ist es auch, von dem distal gesetzten Orthoimplantat **42** Drähte **61** direkt an einen Zahn - hier der 2. Molar - zu legen, um direkt mittels der Vorspannung im Draht **61** auf den Zahn im Sinne einer Positionskorrektur einzuwirken.

Aufgrund des verbesserten Sitzes der Orthoimplantate gegenüber Vorgängerprodukten und Verkleinerung ihrer Abmasse, ist es auch möglich, die Orthoimplantate dort im Mundraum einzusetzen, wo nur wenig Platz zur Verfügung steht, also auch im retromolaren Bereich des Unterkiefers.

### Figuren 6A bis 6C

Für das Aufsetzen der Klemmkappen auf die Orthoimplantate ist ein spezieller Schraubendreher vorgesehen. Dieser besteht aus einem hinten angeordneten Ratschenkopf **72**, einem davon axial wegführenden Stangenteil **73**, wobei die beiden letzteren miteinander fest verbunden sind. An das Stangenteil **73** setzt ebenfalls feststehend der dünnere Schraubendreherschaft **74** an. Am Übergang vom Schraubendreherschaft **74** zum Stangenteil **73** entsteht dadurch eine Anschlagschulter **75**. An der Spitze des Schraubendreherschaftes **74** befindet sich der Schraubendreherkopf **76** mit der passfähigen Kontur zur Ausnehmung **41** im Kopf **40** der Okklusalschraube **38**.

Auf dem Schraubendreherschaft **74** sitzt eine drehbare und längsverschiebliche Hülse **77**, die nur zu Reinigungszwecken abziehbar ist; gleichwohl kann aber der Schraubendreherkopf **76** in das Innere der Hülse **77** gezogen werden. Durch eine Verengung in der Hülse **77** verklemmen sich im zurückgezogenen Zustand die Hülse mit dem Schraubendreherkopf **76**. Die Hülse **77** weist vorn eine Verdickung **78** auf, durch die sich eine axiale Bohrung **79** erstreckt, in welcher der Schraubendreherkopf **76** hin- und herfahren bzw. sich drehen kann. An der Eintrittsseite der Hülse **77** ist eine flachzylindrische Ausnehmung **80** eingearbeitet. In die Ausnehmung **80** ist die Klemmkappe **51** mit eingesetzter Okklusalschraube **38** mit zugewandter Oberseite **52** einsteckbar.

Das Fixieren einer Klemmkappe **51** auf einem Orthoimplantat **42** mittels des Schraubendrehers geschieht auf folgende Weise:

### Figur 6A

Um die Klemmkappe **51** und die Okklusalschraube **38** sicher zu fassen, steckt man diese Kombination in die Ausnehmung **80** der Hülse **77**.

### Figur 6B

Nun wird die Hülse **77** in von der Anschlagschulter **75** wegweisender Richtung gezogen bis die Verklemmung der Hülse **77** auf dem Schraubendreherschaft **74** eintritt. Man hat jetzt eine starre Einheit und kann die Klemmkappe **51** mit der Drahtnut **58** und dem eingefädelten Draht **61** ausrichten.

### Figur 6C

Schliesslich drückt man den Schraubendreherschaft **74** in die Hülse **77** hinein. Hierdurch löst sich die Verklemmung zwischen der Hülse **77** und dem Schraubendreherschaft **74** und der Schraubendreherkopf **76** greift in die Ausnehmung **41** der Okklusalschraube **38** ein, so dass die Verschraubung erfolgen kann. Die Okklusalschraube **38** schraubt sich in das Innengewinde-Sackloch **14** des Orthoimplantates **44** ein. Ist das Einschrauben beendet, zieht man den Schraubendreher von der angeschraubten und positionierten Klemmkappe **51** ab, unter der der Draht **61** fixiert ist.

Der Löse- bzw. Justiervorgang der Klemmkappe **51** mit dem Schraubendreher geschieht auf analoge Weise. Im Schraubenkopf **40** der Okklusalschraube **38** sind als Ausnehmung **41** z.B. ein Kreuzschlitz oder ein Innensechskant denkbar, wobei der Schraubendreherkopf entsprechend passfähig gestaltet ist.

## Patentansprüche

1. Implantat-System für die Orthodontie mit einem Orthoimplantat (**42**), welches einen Gewindeteil (**5**) mit einem Aussengewinde (**6**) besitzt, das zum Einsetzen in einen Knochen (**60**) bestimmt ist; wobei
a) das Gewindeteil (**5**) Gewindezähne (**62**) und durch Schneidausnehmungen (**10**) gebildete Schnittkanten (**11**) aufweist,
b) das Orthoimplantat (**42**) in der Kopfzone komplementär zu einer aufsetzund fixierbaren Klemmkappe (**51**) gestaltet ist, und
c) die Klemmkappe (**51**) mit einer Okklusalschraube (**38**) auf das Orthoimplantat (**42**) geschraubt ist,
dadurch gekennzeichnet, dass
d) die Klemmkappe (**51**) auf der dem Orthoimplantat (**42**) zugewandten Seite eine tangentiale Drahtnut (**58**) aufweist, und
e) zwischen dem orthoimplantat (**42**) und der Klemmkappe (**51**) ein zugeführter orthodontischer draht (**61**) einklemmbar ist.

2. Implantat-System nach Anspruch 1, dadurch gekennzeichnet, dass am Orthoimplantat (**42**) die zwischen benachbarten Gewindezähnen (**62**) verbleibenden Gewindegründe (**63**) zumindest etwa die Breite der Gewindezähne (**62**) am Kerndurchmesser (**dk**) aufweisen.

3. Implantat-System nach Anspruch 1, dadurch gekennzeichnet, dass am Orthoimplantat (**42**)
a) sich die Schneidausnehmungen (**10**) von der Basisfläche (**9**) des Gewindeteiles (**5**) axial und peripher um das Gewindeteil (**5**) erstrecken; und
b) beim Eindrehen in den Knochen (**60**) sich von den Schnittkanten (**11**) abgeschabte Knochensubstanz in den Schneidausnehmungen (**10**) sammelt und weitere Knochensubstanz in einem Verdichtungsbereich (**65**) und den entstandenen Innengewindegang komprimiert.
4. Implantat-System nach einem der Ansprüche 1 bis 3, dadurch gekennzeichnet, dass zur Zahnstellungskorrektur am Orthoimplantat (**42**)
a) orthodontischer Draht (**61**) einseitig oder beiderseits abgehend fixierbar ist, welcher dazu bestimmt ist, sich hin zum zu verschiebenden Zahn oder zu einer zu verschiebenden Zahngruppe zu erstrecken; und
b) zusätzlich eine Feder (**69**) fixierbar ist.
5. Implantat-System nach Anspruch 1 mit einem Schraubendreher zum Auf- und Abschrauben sowie Positionieren der Klemmkappe (**51**), dadurch gekennzeichnet, dass
a) der Schraubendreher eine im Verhältnis zu einem Ratschenkopf (**72**) drehbare und auf einem Schraubendreherschaft (**74**) verschiebbare, jedoch auch fixierbare Hälse (**77**) aufweist, die an ihrer Eintrittsseite die Klemmkappe (**51**) mit eingesetzter Okklusalschraube (**38**) und zugewandter Oberseite (**52**) aufnimmt;
b) axial durch die Hälse (**77**) ein Schraubendreherkopf (**76**) der Ausnehmung (**41**) in der Okklusalschraube (**38**) zuführbar ist; und
c) der Schraubendreherkopf (**76**) und die Ausnehmung (**41**) zueinander komplementäre Konturen besitzen.
6. Implantat-System nach Anspruch 5, dadurch gekennzeichnet, dass
a) am Schraubendreher der Ratschenkopf (**72**) mit dem Schraubendreherschaft (**74**) und dem Schraubendreherkopf (**76**) eine sich axial erstreckende starre Einheit ergeben; und
b) die gegenseitige Längsverschiebbarkeit mit der Hälse (**77**) durch beiderseitige Anschläge begrenzt ist.

## Claims

1. Implant system for orthodontics with an orthoimplant (**42**) which has a threaded part (**5**) having an external screw thread (**6**) designated to insert into a bone (**60**), wherein
a) the threaded part (**5**) is provided with thread crests (**62**) and cutting edges (**11**) formed by cutting recesses (**10**),
b) the orthoimplant (**42**) is, in the head zone, configured complementarily with a fit-on and fixable clamping cap (**51**), and
c) the clamping cap (**51**) is screwed onto the orthoimplant (**42**) using an occlusal screw (**38**), characterised in that
d) the clamping cap (**51**) has at its side faced to the orthoimplant (**42**) a tangential wire groove (**58**), and
e) a tangentially led orthodontic wire (**61**) can be clamped between the orthoimplant (**42**) and the clamping cap (**51**).

2. Implant system according to Claim 1, characterised in that the thread roots (**63**) remaining between neighbouring thread crests (**62**) of the orthoimplant (**42**) have at least approximately the width of the thread crests (**62**) at the core diameter (**dk**).

3. Implant system according to Claim 1, characterised in that at the orthoimplant (**42**)
a) the cutting recesses (**10**) extend axially from the base surface (**9**) of the threaded part (**5**) and peripherally around the threaded part (**5**); and
b) on screwing into the bone (**60**), bone substance abraded by the cutting edges (**11**) collects in the cutting recesses (**10**) and compresses further bone substance, in a compression region (**65**), and the resulting internal thread flight.

4. Implant system according to one of the Claims 1 to 3, characterised in that for a tooth alignment correction at the orthoimplant (**42**)
a) an orthodontic wire (**61**) can be fixed outgoing on one or both sides of the orthoimplant (**42**) is designated to extend towards a tooth or a tooth group to be displaced; and
b) additional a spring (**69**) can be fixed.

5. Implant system according to Claim 1 with a screwdriver for screwing on and unscrewing as well as positioning the clamping cap (**51**), characterised in that
a) the screwdriver has a socket (**77**) that can be rotated in ratio with a ratchet head (**72**) and is displaceable but also fixable on a screwdriver shaft (**74**), which socket (**77**) receives at its entry side the clamping cap (**51**) with filled occlusal screw (**38**) and facing upper side (**52**);
b) it being possible to feed a screwdriver head (**76**) to the recess (**41**) in the occlusal screw (**38**) axially through the socket (**77**); and
c) the screwdriver head (**76**) and the recess (**41**) having mutually complementary contours.

6. Implant system according to Claim 5, characterised in that
a) the ratchet head (**72**) with the screwdriver shaft (**74**) and the screwdriver head (**76**) produce an axially extending rigid unit at the screwdriver; and
b) the reciprocal longitudinal displaceability with the socket (**77**) is limited by catches on both sides.

## Revendications

1. Système d'implant pour l'orthodontie, avec un orthoimplant (42), qui possède une partie filetée (5) avec un filetage externe (6) qui sert à l'insertion dans un os (60); dans lequel
a) la partie filetée (5) présente des sommets de filet (62) et des arêtes de coupe (11) formées par des évidements de coupe (10),
b) l'orthoimplant (42) a une forme complémentaire, dans la région de la tête, à une coiffe d'ancrage (51) susceptible d'être posée et fixée, et
c) la coiffe d'ancrage (51) est vissée sur l'orthoimplant (42) avec une vis occlusale (38),
caractérisé en ce que
d) la coiffe d'ancrage (51) présente une gorge tangentielle (58) pour un fil métallique, sur le côté tourné vers l'orthoimplant (42), et
e) un fil métallique orthodontique (61) amené peut être coincé entre l'orthoimplant (42) et la coiffe d'ancrage (51).

2. Système d'implant selon la revendication 1, caractérisé en ce que les fonds de filet (63) restant entre des sommets de filet voisins (62) sur l'orthoimplant (42) présentent au moins environ la largeur des sommets de filet (62) au niveau du diamètre du noyau (dk).

3. Système d'implant selon la revendication 1, caractérisé en ce que sur l'orthoimplant (42)
a) les évidements de coupe (10) s'étendent depuis la surface de base (9) de la partie filetée (5) axialement et circonférentiellement autour de la partie filetée (5); et
b) lors du vissage dans l'os (60), de la substance osseuse raclée par les arêtes de coupe (11) s'accumule dans les évidements de coupe (10) et de la substance osseuse supplémentaire se comprime dans une zone de compression (65) et le pas du filetage interne ainsi créé.

4. Système d'implant selon l'une quelconque des revendications 1 à 3, caractérisé en ce que pour corriger la position des dents,
a) un fil métallique orthodontique (61) peut être fixé sur l'orthoimplant (42), partant vers un côté ou les deux côtés, lequel est prévu pour s'étendre vers une dent à déplacer ou vers un groupe de dents à déplacer; et
b) un ressort (69) peut en outre être fixé sur l'orthoimplant (42).

5. Système d'implant selon la revendication 1, avec un tournevis pour le vissage et le dévissage ainsi que pour le positionnement de la coiffe d'ancrage (51), caractérisé en ce que
a) le tournevis comprend une douille (77) rotative par rapport à une tête de cliquet (72), et susceptible d'être déplacée, mais aussi fixée, sur une tige de tournevis (74), la douille recevant, sur son côté d'entrée, la coiffe d'ancrage (51) avec la vis occlusale (38) insérée et sa partie supérieure (52) tournée vers elle;
b) une tête de tournevis (76) peut être amenée axialement à travers la douille (77) dans l'évidement (41) dans la vis occlusale (38); et
c) la tête de tournevis (76) et l'évidement (41) ont des contours complémentaires.

6. Système d'implant selon la revendication 5, caractérisé en ce que
a) sur le tournevis, la tête de cliquet (72), la tige de tournevis (74) et la tête de tournevis (76) forment une unité rigide s'étendant axialement; et
b) la capabilité de déplacement longitudinal réciproque par rapport à la douille (77) est limitée par des butées sur les deux côtés.
